# EUROPEAN PATENT APPLICATION

(11) **EP 4 215 111 A2**
(43) Date of publication of application: **26.07.2023**
(21) Application number: 23162195.4
(22) Date of filing: 04.03.2014
(51) Int. Cl.: A61B 5/07, A61B 5/20, A61B 5/00, A61M 31/00, A61B 5/145, A61F 6/14

(54) **VAGINAL DRUG DELIVERY AND/OR DIAGNOSTIC SYSTEM**

(30) Priority: 08.03.2013 EP 13158451
(62) Divisional of application: 14707785.3
(71) Applicant: Li Galli B.V., 2514 AC Den Haag (NL)
(72) Inventor: De Laat, Wilhelmus Nicolaas Gerardus Maria, Den Haag (NL)
(74) Representative: Arnold & Siedsma

(57) **Abstract**

The present invention relates to a vaginal drug delivery and/or diagnostic system, which is connected to a positioning member configured for placement within the vagina. The system comprises an electronic capsule comprising a drug reservoir and means for dispensing the drug from the reservoir into the vagina. The positioning member is for example eight-shaped, pear-shaped or dolphin-shaped and is preferably provided with friction-enhancing means to keep the device in place. The system is particularly useful for systemic administration of drugs and hormones.

## Description

The present invention relates to a vaginal drug delivery and/or diagnostic system.

Drug delivery systems for placement in the vagina are well known in the art. Such devices are usually ring-shaped and are formed of polymers that are loaded with one or more drugs, in particular steroid hormones, that are released in a pre-defined pattern. To obtain a desired release pattern, such rings may be provided with different compartments or different layers of different materials comprising different drugs or drugs in different concentrations.

A well-known example is the Nuvaring^{®} (EP-0876815), that consists of a thermoplastic polymer core and a skin covering the core, wherein an estrogenic compound and a progestogenic compound are dissolved in the polymer core in a concentration that leads to the desired release pattern.

US-4,237,885 relates to a tube or coil of polymeric material that is divided into portions that are separated by means of spacers, in which each of the portions contains a different active substance and in which the ends of the tube are joined together to form a ring.

US-4,596,576 discloses a two-compartment vaginal ring, wherein each compartment contains a different active substance. In order to avoid mixing of the various active substances leading to fluctuations in the release ratio, the compartments had to be isolated from each other by means of stoppers.

Although the above described release systems which release one or more active substances in a substantially constant ratio over a prolonged period of time have been proven useful for certain applications in the field of contraception, hormone replacement therapy or local therapy, they have the disadvantage that their release pattern is pre-programmed and cannot be adapted during the treatment period. More specifically, they do not allow for a cyclic release pattern or release during a specific number of hours of the day, days of the month nor for release starting at certain points of time. For some diseases or medication regimes, such as Dopa medication for the treatment of Parkinson disease, even frequent oral dosing does not address the disease optimally.

Moreover, it was found that not all drugs and hormones can be dissolved in the pharmaceutically acceptable polymers that are used in the prior art devices.

Furthermore, in the context of personalized medicine there is a need for interactive medication adapted to the specific needs of a patient, or adjusted for the specific weight or gender of the patient. Finally, a substantial number of compounds is currently not suited or oral administration (due to poor absorption, short half life and other *tractus digestivus* related reasons) and highly inconvenient via invasive parenteral administration (such as contraception via daily injection).

It is the object of the present invention to provide a vaginal drug delivery system that is in particular useful for systemic drug delivery and obviates the above described disadvantages, is also suitable for diagnosis and provides the possibility of interactive and personalized medicine. The invention has furthermore as an object to enable the use of a substantial number of hitherto unsuited compounds in human and veterinary medicine, in particular in human.

The invention therefore relates to a vaginal drug delivery and/or diagnostic system, comprising an electronic capsule, which is connected to a positioning member configured for placement within the vagina. The system may also comprise more than one capsule, in particular two or three capsules. When in this application reference is made to "a capsule" or "one capsule" this term also refers to "at least one capsule" or "one or more capsules".

The electronic capsule comprises one or more components selected from drug reservoirs, diagnostic means and means for dispensing the drug from the reservoir into the vagina. In addition to these components other components performing other functions may be included. The capsule may comprise various combinations of these components and/or the system may comprise multiple capsules that perform one or more of the desired functions and comprise the corresponding components. A drug reservoir can be either incorporated into a capsule or can be used as such as a separate entity functioning as stock reservoir for the drug and/or as release reservoir for dispensing the drug.

The vaginal system of the invention allows the delivery of pharmaceutically active compounds that work systemically.

When the system is intended or also intended for diagnosis the capsule comprises diagnostic means instead of or in addition to the drug reservoir and the dispensing means.

The positioning member has preferably a substantially closed peripheral shape. The member is preferably completely closed to improve its dimensional stability but members that are not completely closed are also part of this invention.

The positioning member can have different shapes. In one embodiment, the substantially closed peripheral shape is formed by two parts, one being larger than the other. The part with the smaller diameter is for placement in the distal part of the vagina, whereas the larger part is located closer to the proximal part of the vagina, i.e. closer to the introitus. Preferably, each part of the positioning member defines a plane, the plane defined by the first part being offset with respect to the plane defined by the second part. This means that the two parts of the member do not or need not lie in the same plane.

In a preferred embodiment, the positioning member is eight-shaped. The position of the capsule in the positioning member can vary but the capsule is preferably located in between the two parts connecting two ring structures to ensure a position of the capsule closest to the *cervix uteri,* which is the exit of/entrance to the *cavum uteri.*

The capsule is suitably an integral part of the positioning member, i.e. incorporated in the member structure, but can also be connected thereto in a more loose way.

In an alternative embodiment, the positioning member is pear-shaped. Suitably, when in use, the smaller part of the pear shape is located at the distal end of the vagina and the larger part at the proximal end. Also in this configuration both parts of the positioning member define a plane, wherein the plane defined by the first part is offset with respect to the plane defined by the second part.

In the pear-shaped embodiment, the capsule is suitably located at the transition between the two planes. The system may comprise more than one electronic capsule. In the pear shape, the system has at least one and suitably two capsules. The inclusion of two capsules which are both located at the transition between the two planes leads to a symmetrical configuration of the system.

In a further embodiment the positioning member is configured as a flat disc. The two parts of the positioning member are then not in different planes but in the same plane. This shape lies parallel to or in the same plane as the upper and lower vaginal wall thus enabling a maximal contact surface for exchanging diagnostic parameters and/or release of medication.

A further embodiment of the invention provides the system in a fish or dolphin shape. In a preferred embodiment of this shape the larger part is not open but closed and contains the different components of the electronic capsule. Suitably a ring structure surrounds the vaginal system for extra stiffness. The ring structure may be more flexible where the open and closed parts meet to allow adaptation to individual vaginal anatomy.

The drugs can be released through pores in the wall of the rim or frame of the system through specialized outlets, etc. In a further embodiment, the positioning member is at its surface provided with contact and/or absorption improving means, such as protuberances. In one embodiment the protuberances resemble the form of microvilli. In another embodiment the positioning member may be partially or completely covered by specific nano-structures, which by interaction with the vaginal wall, can temporarily increase its permeability to the released substance(s).

The system of the invention should not be expelled from the vagina when in use. The positioning member can be provided with various expulsion inhibiting means either alone or in combination.

In one embodiment, the system is clamped against the lateral vaginal walls. Such a positioning member is suitably ring-shaped for optimal clamping.

In a preferred embodiment, the positioning member is provided with friction enhancing means. The friction enhancing means should be compatible with the vagina and are chosen such that the do not cause any damage or irritation when the drug delivery and/or diagnostic system is in use. The friction enhancing means are suitably formed by a rough surface and are preferably formed by a surface mimicking the vaginal rugae. This means that the surface has the same shape as the rugae or has a complementary shape. The rough surface can be jagged, serrated, dentate, ridged, notched, incised, toothed or otherwise shaped.

These friction enhancing means assist in stabilizing the drug delivery and/or diagnostic system in the desired position and orientation in the vagina. The friction enhancing means lead to a better contact between the system and the vaginal wall thus also allowing better transfer of the drug. Furthermore, expulsion of the ring is prevented.

The friction enhancing means can be located in a more flexible part of the positioning member preventing displacement under pressure.

When the vaginal system is not flat but curved or vaulted in the lower portion of the positioning member vacuum fixation to the vaginal wall prevents displacement. When the curving or vaulting is at the top of the positioning member, vacuum fixation in the fornix posterior is possible.

The vaginal drug delivery system of the invention is preferably configured such that at least the part of the positioning member closest to the capsule can distribute the drug continuously or intermittently, as programmed, over a larger surface of the positioning member to ensure an optimal absorption by the vaginal mucosa.

In a further embodiment, the positioning member is provided with a reservoir, in particular a release reservoir. The reservoir may be formed by a hollow part of the member or is a separate compartment.

This reservoir can be suitably into contact with the capsule and can be used for receiving at least part of the drug after it is dispensed from the capsule. Suitably, the wall of this reservoir is permeable to the contents of the reservoir. Upon contact with the surroundings the drug is released through the permeable wall of the reservoir. This way the drug is dispensed over a larger surface thus allowing close and increased contact with the vaginal wall. The permeable reservoir wall can be tailored such that a continuous and gradual release of the drug is obtained. Alternatively, the release can be intermittent when the drug is only periodically released into the reservoir. This can for example be useful in contraceptive regimens wherein monthly discontinuation of the hormone administration is desirable in order to induce a withdrawal bleeding.

The reservoir in the positioning member may be used for receiving the substance dispensed by the capsule but may also have a different content.

In one embodiment, the reservoir contains a fluid that can be mixed with the contents of the capsule. The content of the capsule may be a solid compound, such as a powder, that is dissolved in the fluid or may be a fluid itself. The mixing can be done by the pump of the electronic capsule. The mixture or solution obtained can then be stored in another hollow part of the positioning member and released therefrom at the desired time and at the desired rate. Suitably this release is by means of diffusion.

The reservoir may also contain other substances, such as absorption promoting additives that increase the capacity of the vaginal mucosa to absorb the medication at the place where the medication is released from the capsule or the positioning member. If the absorption promoting additives are for increasing the absorption of a substance released from the capsule, the reservoir containing these additives is suitably located close to or adjacent to the capsule.

The electronic capsule that is preferably used in combination with the positioning member of the invention is the device as described in WO2011/039680. This capsule comprises a reservoir for storing a substance to be administered that communicates with the environment surrounding the capsule via a dispensing hole. The reservoir compartment is separated from a motor compartment that houses the pump. Suitably, the capsule also has an electronics compartment.

The capsule may further be provided with one or more sensors or a so-called "lab-on-a-chip" for monitoring various parameters of the patient, such as, but not limited to, cervical mucus qualities, hormone levels, glucose levels, temperature, pH etc. The sensors/chip could perform health screening tests, monitor biochemical parameters, perform a search for oncological cells (such as cancer of ovaries, endometrium or cervix) or infections like vaginitis or other physiological, such as, but not limited to, neurological, functions, etc. This could be done only once or repeatedly and continuously or intermittently.

The sensors may also be comprised in the positioning member. The capsule can suitably communicate with a receiver system outside the body via a wireless link. The receiver system can for example record the data that are measured by the sensors and adapt the release pattern of the substance contained in the reservoir. This way, the entire system becomes interactive. Administration of drugs can be completely personalized by adapting the release of the drug to the values of various parameters measured by the sensors.

Alternatively, the sensor can communicate with the dispensing means and directly control the release of the active substance therefrom based on the measurements made by the sensor. The sensor can suitably measure a particular parameter and optionally compare the measured value with a desired value for the parameter and adapt the release of the active substance thereto. In one embodiment it is for example possible to use a blood glucose sensor for measuring the sugar levels in the blood of a patient. Based on the blood glucose level measured the release of insulin from a reservoir in the same or in a separate capsule is controlled. In a further embodiment not only insulin but also glucagon can be dispensed to either decrease or increase the blood glucose level of the patient.

In a further embodiment the vaginal system can be used to measure fertility parameters in assisted reproduction treatments. By measuring FSH, LH and 170-estradiol the correct moment for particular steps in the treatment can be determined. Such steps are for example pregnyl administration, moment of insemination, time of harvest of the follicles. Suitably, the measured parameters are transmitted to the doctor who can determine the timing of the next step in the treatment. In this embodiment the system communicates with the outside world and transmits information to the doctor.

A further use of the vaginal system of the invention relates to measuring the concentration of a particular drug in the blood. Based on the measured concentration in comparison to the desired concentration the release of the drug can be controlled or adapted. This can be done by the system, either within a capsule or between capsules, without interference from outside. This use is in particular suitable for the treatment with Dopa in Parkinson disease.

The above and other uses lead to highly personalized medicine that is completely adapted to the specific needs of an individual.

In this patent application the electronic device that comprises the drug storage and drug delivery means and/or the diagnostic means is called a "capsule". The term capsule implies that the device has a flush exterior without any protrusions or projections. Such a shape is disclosed in WO2011/039680 and is a preferred embodiment. However, the system of the invention can also comprise differently shaped electronic devices and the separate functions of the device can even be incorporated in separate locations in the positioning member. All such other embodiments are to be encompassed by the term "capsule". The word "capsule" thus refers to devices that perform one or more functions in the vaginal system.

In a preferred embodiment the positioning member is configured such that it causes no lesions to the vaginal epithelium and cervix at insertion or removal or use of the vaginal system of the invention. The materials used in the vaginal system are of an approved medical grade and are preferably non irritating and non allergic.

The configuration is suitably such that there is no risk of breakage or disintegration of electronic parts into the vagina.

In a preferred embodiment, the vaginal system comprises a built-in prevention of erroneous dose delivery.

The diagnostic functions of the vaginal system relate in particular to temperature monitoring, LH, FSH monitoring, monitoring of various steroid hormones, pH measurement, viscosity measurement, protein analysis, nucleic acid analysis, in particular DNA compounds, detection of signaling molecules (e.g. cytokines), or other physiological, like neurological, functions, etc.

When the vaginal system is intended for drug delivery it comprises a drug storage space. In case the storage space is located remote from the release site, the vaginal system may include a transport system from the storage space to the drug release site. The release site is suitably a membrane or micro-pore surface at the drug delivery site. The type of drug delivery can be variable, continuous, interrupted, single drug or any other type of delivery.

The drug is suitably delivered in a fluid solution, in a gel composition that is optionally adhesive or a foam composition. The composition of the delivery vehicle (solution, gel, foam or other fluid) is suitably selected such that the vaginal ecology (e.g. pH) is restored or protected.

The electronic capsule when provided with diagnostic sensors suitably comprises an internal connection of the electronic system to the diagnostic sensors and to the drug release parts (storage, transport, contact surface). The capsule then also comprises means for analyzing the diagnostic parameters. Suitably, the diagnostic data are stored in the capsule on storage means and/or transmitted to external data equipment. The data can be transmitted wirelessly.

It is possible to control the function of the electronic capsule via a receiver for external control of function.

Suitably, the capsule is embedded in a protected housing and comprises a system of alert signaling and automatically switching off at malfunction.

The system can be placed intra-vaginally for a desired period of time. For contraception this is suitably 4 weeks but longer or shorter periods can also be envisaged.

The system is designed such that intercourse is possible while the system is in use. This means for example that the system should not cause any lesions at the side of the partner at intercourse, that there should be no adverse effect of the drug delivered by the system on the partner and that there should be no expulsion of the system. Moreover, intercourse should not induce any unwanted discharge of the drug during intercourse. Furthermore, the position of the system in the vagina is such that it allows correct monitoring. The configuration of the systems as described herein meet these requirements.

The configuration of the systems as described herein are easily accepted by the user, can be easily inserted and removed by the user. Suitably the system is packaged with clear instructions for insertion and removal. An additional insertion device for inserting the system in the correct position is also part of this invention. The system can be removed with a finger or an especially configured removal device.

The shape of the systems described herein lead to comfort at use, i.e. does not lead to pain or irritation.

In a preferred embodiment the system can be remotely controlled.

The drug delivery and/or diagnostic system of the invention can be used in the diagnosis of fertility problems cycle analysis, chronic infections, autoimmune diseases and for treatment thereof. Medication can furthermore be directed at atrophy of the vagina, endometriosis, etc., but also at other, more distant bodily malfunctions.

The drug delivery and/or diagnostic system of the invention is very useful for medication having a favourable dose-effect profile at vaginal administration since the dose can then be lower. The system is also very useful for medication for which a first-pass effect through the liver should be avoided.

The possibility of continuous administration during 24 hours/day will result in stable systemic serum levels of the administered compound and will be beneficial for the therapeutic result, certainly if even frequent oral dosing does not address the disease optimally (e.g. in the case of Dopa medication for the treatment of Parkinson disease). Moreover, the potential of the system to deliver drugs "on demand" has advantages in, for instance but not limited to, insulin-dependent diabetes, Parkinson's disease (so-called "on/off' episodes), etc.

Combining the diagnostic potential of the vaginal system (in particular the embodiment with two capsules) with the drug delivery application may results in continuous or intermittent dosing and continuous adjustment of the administered compound based on the parameters measured by the diagnostic vaginal system (e.g. insulin and glucose levels in diabetes).

Another application of the system is pH regulation in the vagina.

In a particular embodiment of the invention, the vaginal system of the invention comprises a sensor for measuring the blood glucose level, means for dispensing insulin, optional means for dispensing glucagon and means for determining the amount of insulin and optionally glucagon to be dispensed on the basis of the blood glucose level measured and a desired blood glucose level. Sensors for transcutaneous measurement of glucose levels in the blood are known in the art and can be used in the system of the invention.

Suitably, at least one component selected from the sensor, the dispensing means and the determining means is located in at least one electronic capsule. Other configurations are also possible.

In a further embodiment, the vaginal system comprises a sensor for measuring the level of a drug or hormone in blood, means for dispensing drug or hormone and means for determining the amount of drug or hormone to be dispensed on the basis of the level of the drug or hormone measured and a desired drug or hormone level. In this embodiment the system of the invention is used as a companion diagnostic.

In another embodiment, the vaginal system is for diagnosis and comprises a sensor for fertility parameters and means for transmitting measurement information generated by the sensor to a receiver, in particular an external receiver.

The present invention will be further illustrated in the figures that follow and that are not intended to limit the invention in any way. These figures show:
**Fig. 1****:** A partially cut-away perspective view of the eight-shaped drug delivery and/or diagnostic system of the invention located in the vagina.
**Fig. 2****:** Top views of the eight-shaped vaginal system.
**Fig. 3****:** A partially cut-away perspective view of the pear-shaped drug delivery and diagnostic system of the invention located in the vagina.
**Fig. 4****:** Different views of the pear-shaped system.
**Fig. 5****:** A partially cut-away perspective view of the dolphin-shaped drug delivery and diagnostic system of the invention located in the vagina.
**Fig. 6****:** Different views of the dolphin-shaped system.

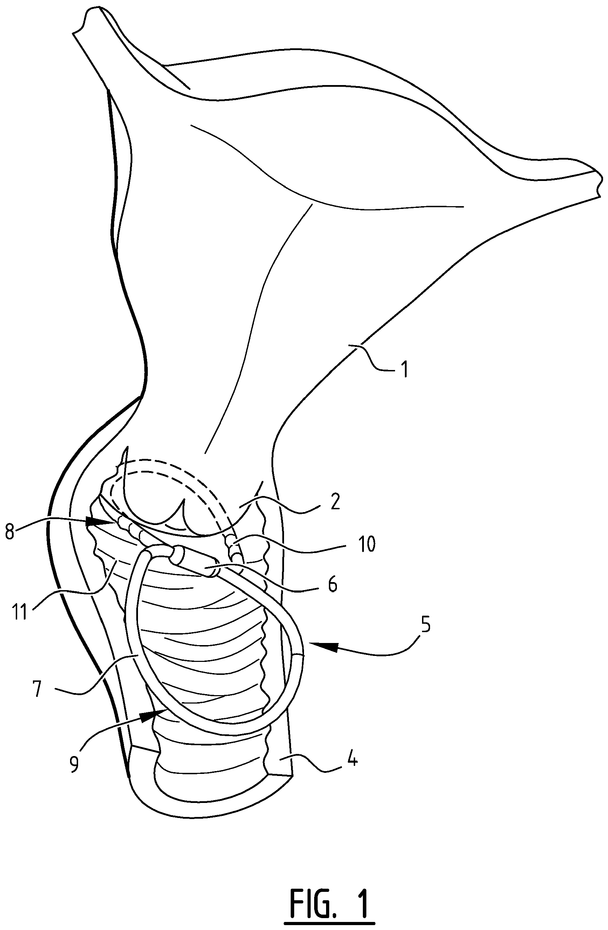

**Fig. 1** shows a partially cut-away perspective view of the uterus 1, cervix 2 and vagina 4 of a female subject. The eight-shaped drug delivery and/or diagnostic system of the invention 5 is located in the vagina. The system comprises an electronic capsule 6 that is comprised in a positioning member 7. The positioning member 7 comprises two separate parts 8 and 9 that are connected. The electronic capsule 6 is located in between the parts 8 and 9. The smaller part 8 surrounds the cervix 2 and comprises rough surfaces 10 that mimic the vaginal rugae 11 and keep the vaginal system in place.

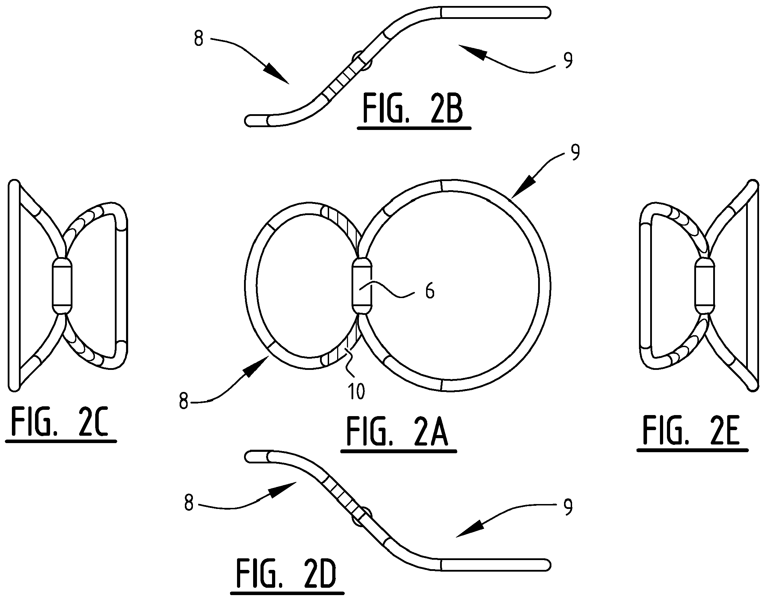

**Fig. 2** shows different views of the eight-shaped drug delivery and/or diagnostic system of the invention 5. **Fig. 2A** is a top or plan view that shows the eight-shape and that the part 8 is smaller than part 9. The left and right side views of **Fig. 2B** and **Fig. 2D** and rear and front views. **Fig. 2E** and **Fig. 2C** show that the two parts 8 and 9 are in different planes.

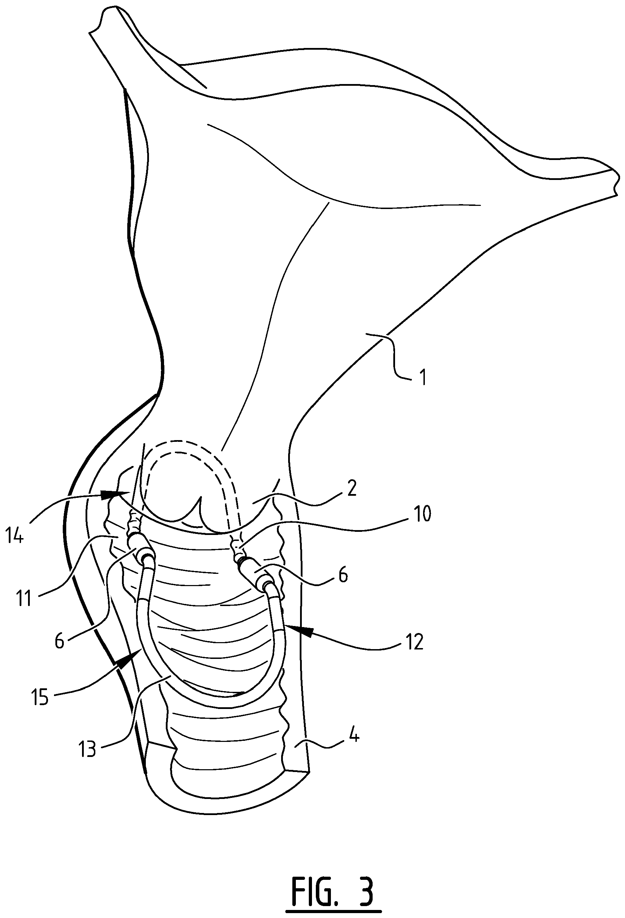

**Fig. 3** shows a partially cut-away perspective view of the uterus 1, cervix 2 and vagina 4 of a female subject. The pear-shaped drug delivery and/or diagnostic system of the invention 12 is located in the vagina. The system comprises two electronic capsules 6 that are comprised in a positioning member 13. The positioning member 13 comprises two separate parts 14 and 15 that are connected. The two electronic capsules 6 are located at the transition between the two parts 14 and 15. The smaller part 14 partially surrounds the cervix 2 and comprises rough surfaces 10 that mimic the vaginal rugae 11 and keep the vaginal system in place.

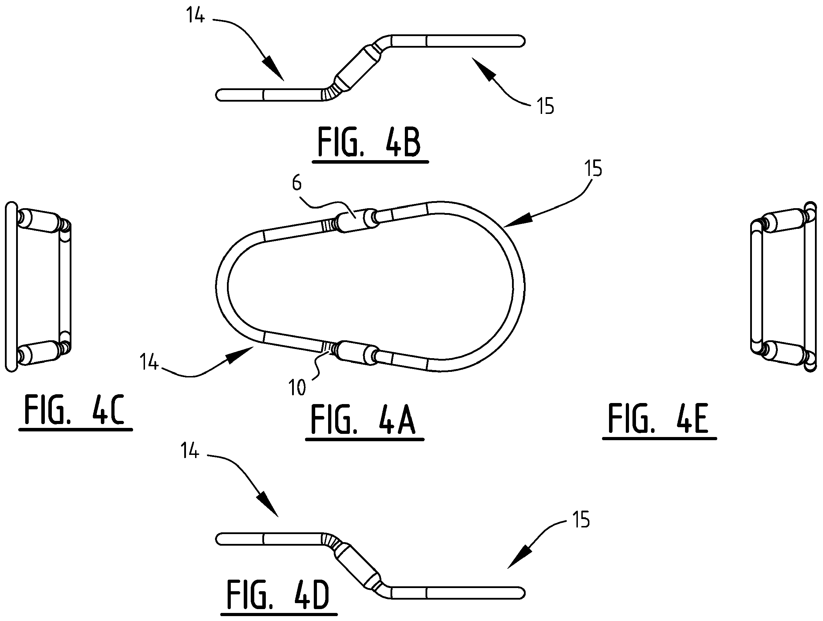

**Fig. 4** shows different views of the pear-shaped drug delivery and/or diagnostic system of the invention 12. **Fig. 4A** is a top or plan view that shows the pear-shape and the location of the two capsules 6. The left and right side views of **Fig. 4B** and **Fig. 4D** and rear and front views **Fig. 4E** and **Fig. 4C** show that the two parts 14 and 15 are in different planes.

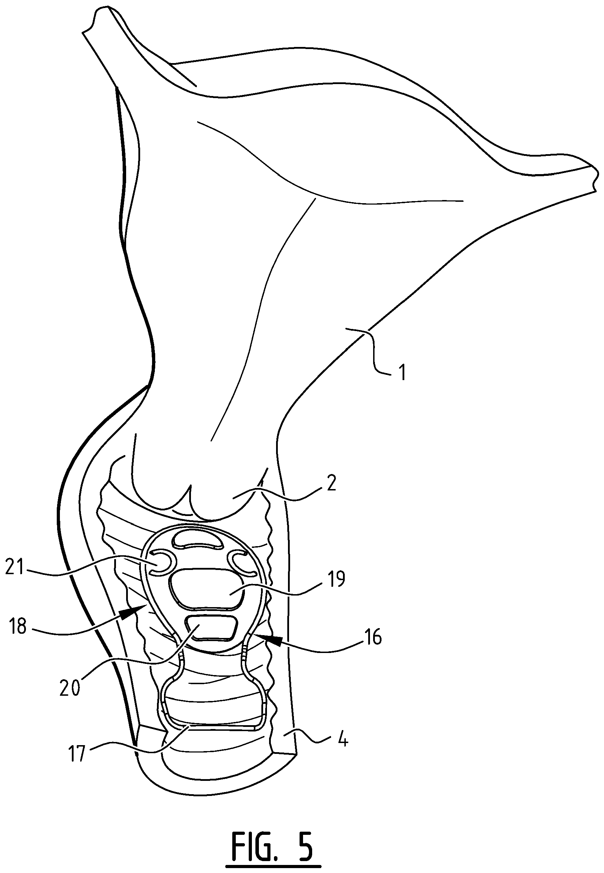

**Fig. 5** shows a partially cut-away perspective view of the uterus 1, cervix 2 and vagina 4 of a female subject. The dolphin- or fish-shaped drug delivery and/or diagnostic system of the invention 16 is located in the vagina 4. The tail part of the fish is closest to the entrance of the vagina and forms a loop 17 for easy removal of the system. The other part 18 comprises all components of the electronic capsule, such as a reservoir 19, a sensor 20 for diagnostic measurements, electronic components 21. The shape may be flat or slightly vaulted at either or both end.

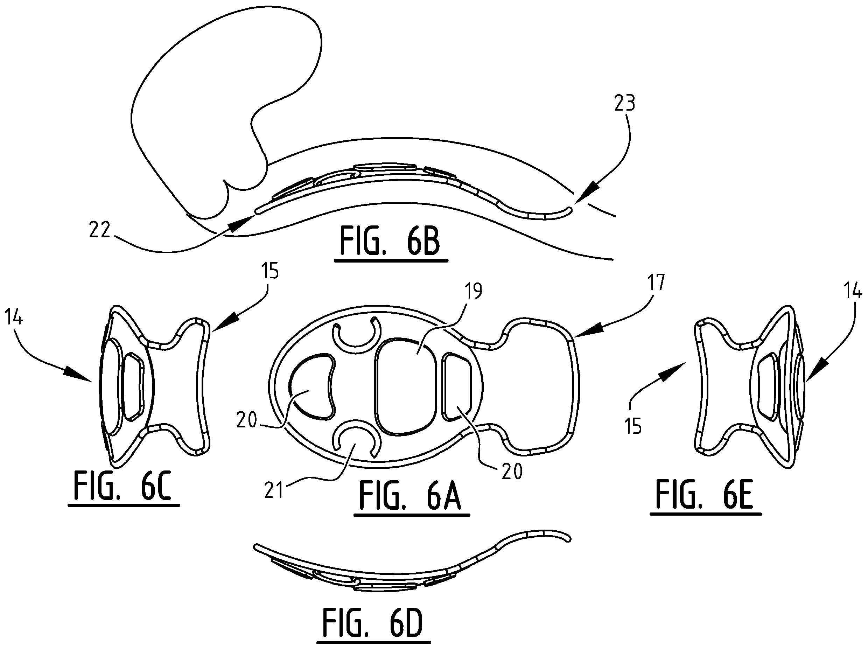

**Fig. 6A** is a top or plan view that shows the dolphin-shape and the location of the two capsules 6. The left and right side views of **Fig. 6B** and **Fig. 6D** and rear and front views **Fig. 6E** and **Fig. 6C** show that the two parts 14 and 15 are in different planes.

**Fig. 6B** shows the dolphin-shaped system 16 from the side and located in the vagina 4. In this embodiment, the system is essentially flat but slightly vaulted at both ends 22 and 23 to allow for better adaptation to the vaginal anatomy.

## Claims

1. Vaginal drug delivery and/or diagnostic system, comprising an electronic capsule, which is connected to a positioning member configured for placement within the vagina.

2. The vaginal system as claimed in claim 1, wherein the electronic capsule comprises one or more components selected from drug reservoirs, diagnostic means and means for dispensing the drug from a reservoir into the vagina.

3. The vaginal drug delivery and/or diagnostic system as claimed in claim 1, wherein the positioning member has a substantially closed peripheral shape, in particular a substantially closed peripheral shape formed by two parts, one being larger than the other more in particular a substantially closed peripheral shape formed by two parts, one being larger than the other wherein each part defines a plane, the plane defined by the first part being offset with respect to the plane defined by the second part.

4. The vaginal drug delivery and/or diagnostic system as claimed in any one of the claims 1-3, wherein the positioning member is eight-shaped or pear-shaped.

5. The vaginal drug delivery and/or diagnostic system as claimed in claim 4, wherein at least one capsule is located in between the two parts when the positioning member is eight-shaped and wherein at least one capsule is located at the transition between the two planes when the positioning member is pear-shaped.

6. The vaginal drug delivery and/or diagnostic system as claimed in any one of the claims 1-5, wherein the positioning member is provided with friction enhancing means, in particular friction enhancing means formed by a surface mimicking vaginal rugae.

7. The vaginal drug delivery and/or diagnostic system as claimed in any one of the claims 1-6, wherein at least the part of the positioning member closest to the capsule is configured for optimal distribution of the drug over a larger surface of the positioning member.

8. The vaginal drug delivery and/or diagnostic system as claimed in any one of the claims 1-6, wherein the positioning member is provided with a reservoir.

9. The vaginal drug delivery and/or diagnostic system as claimed in claim 7 or 8, wherein the wall of the reservoir is permeable to the contents of the reservoir.

10. The vaginal drug delivery and/or diagnostic system as claimed in claim 9, wherein the reservoir is provided with absorption promoting additives.

11. The vaginal drug delivery and/or diagnostic system as claimed in any one of the claims 1 to 10 for use in the systemic delivery of drugs and hormones.

12. The vaginal drug delivery and/or diagnostic system as claimed in any one of the claims 1 to 11, wherein the positioning member is at its surface provided with contact and/or absorption enhancing means, such as protuberances, in particular in the form of microvilli.

13. The vaginal drug delivery and/or diagnostic system as claimed in any one of the claims 1 to 12, comprising a sensor for measuring the blood glucose level, means for dispensing insulin, optional means for dispensing glucagon and means for determining the amount of insulin and optionally glucagon to be dispensed on the basis of the blood glucose level measured and a desired blood glucose level, wherein in particular at least one of the sensor, the dispensing means and the determining means is located in at least one electronic capsule.

14. The vaginal drug delivery and/or diagnostic system as claimed in any one of the claims 1 to 12, comprising a sensor for measuring the level of a drug or hormone in blood, means for dispensing drug or hormone and means for determining the amount of drug or hormone to be dispensed on the basis of the level of the drug or hormone measured and a desired drug or hormone level.

15. The vaginal drug delivery and/or diagnostic system as claimed in any one of the claims 1 to 12, comprising a sensor for fertility parameters and means for transmitting measurement information generated by the sensor to a receiver, in particular an external receiver.
